(19) Europäisches Patentamt
European Patent Office
Office européen des brevets

(11) **EP 2 515 971 B1**

(12) **FASCICULE DE BREVET EUROPEEN**

(45) Date de publication et mention
de la délivrance du brevet:
**09.08.2023  Bulletin 2023/32**

(21) Numéro de dépôt: **10807429.5**

(22) Date de dépôt: **20.12.2010**

(51) Classification Internationale des Brevets (IPC):
**A61M 5/142** *(2006.01)*        **F04B 43/00** *(2006.01)*

(52) Classification Coopérative des Brevets (CPC):
**A61M 5/14224; F04B 43/0054;** A61M 5/14586

(86) Numéro de dépôt international:
**PCT/CH2010/000319**

(87) Numéro de publication internationale:
**WO 2011/094878 (11.08.2011 Gazette 2011/32)**

(54) **POMPE DOSEUSE A MEMBRANE A USAGE MEDICAL**

MEMBRANPUMPE ZUM DOSIEREN FÜR MEDIZINISCHE ANWENDUNGEN

MEMBRANE DOSING PUMP FOR MEDICAL USE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité:  **23.12.2009  CH 19822009**

(43) Date de publication de la demande:
**31.10.2012  Bulletin 2012/44**

(73) Titulaire: **Rochat, Jean-Denis
1272 Genolier (CH)**

(72) Inventeur: **Rochat, Jean-Denis
1272 Genolier (CH)**

(74) Mandataire: **Stona, Daniel
Invention Angels
Rue de Monthoux 55
1201 Genève (CH)**

(56) Documents cités:
**EP-A1- 1 970 081        FR-A5- 2 046 599
US-A1- 2002 004 015**

EP 2 515 971 B1

**Description**

La présente invention se rapporte à un dispositif de pompe doseuse à usage médical comprenant une enceinte de pompage, une membrane de pompage annulaire dont le bord externe est solidaire de ladite enceinte de pompage et dont le bord interne est solidaire d'une partie centrale d'entraînement plus rigide que ladite membrane, susceptible d'être déplacée parallèlement à elle-même entre une position limite de retour et une position limite de déplacement dans sa course d'expulsion et inversement dans sa course d'aspiration, ces deux positions se situant de part et d'autre du plan contenant le bord externe de ladite membrane annulaire, ladite enceinte de pompage comprenant un clapet d'entrée et un clapet de sortie ouverts par une dépression, respectivement par une surpression dans l'enceinte de pompage, consécutives aux déplacements de ladite membrane de pompage annulaire, la course d'aspiration de ladite membrane résultant de l'énergie accumulée par la déformation élastique de la membrane lors de sa course d'expulsion.

On connaît déjà des pompes de ce type. Le document FR 2 046 599 A5 divulgue une pompe ayant la combinaison des caractéristiques selon la préambule de la revendication 1.

La réalisation d'une telle pompe comme pompe doseuse à usage médical, notamment comme pompe de perfusion à usage unique pose plusieurs problèmes que les pompes à membranes connues ne permettent pas de satisfaire.

Cette pompe doit être précise, petite tout en assurant un débit optimum et doit être bon marché du fait qu'elle n'est pas réutilisable. Le clapet de sortie de la pompe doit assurer la sécurité vis-à-vis d'un écoulement libre de liquide sous une certaine pression qui est typiquement celle de la colonne de liquide entre une poche de liquide de perfusion et la pompe, ou celle consécutive à une pression accidentelle exercée sur la poche de liquide. Compte tenu de cette sécurité, la membrane de pompage doit être à même de supporter la pression de travail qui se compose d'une pression d'ouverture du clapet de sortie qui doit rester fermé jusqu'à une pression déterminée par les normes de sécurité, d'une perte de charge dans le conduit aval ainsi qu'une pression d'utilisation en bout de conduit aval.

La membrane de pompage doit être à même d'aspirer le liquide en passant de sa position limite de déplacement à sa position limite de retour en créant une dépression suffisante en présence de la perte de charge dans les conduits en amont et en aval du dispositif de pompage et du seuil de pression du clapet d'entrée.

Cette aspiration doit s'effectuer aussi rapidement que possible pour assurer un débit optimum compte tenu du volume de l'enceinte de pompage. Toutefois la dépression créée par le retour de la membrane ne doit pas être trop forte pour éviter des chocs dans les conduits, la vaporisation du liquide pompé par une chute de pression supérieure à la pression de vapeur du liquide, ainsi que des effets de cavitation.

On pourrait certes relier la membrane de pompage à l'actuateur pour un entraînement bidirectionnel. Toutefois, une telle solution rendrait l'usage unique plus compliqué et donc plus cher à fabriquer et son montage dans le dispositif d'entraînement plus difficile à réaliser. Ce mode d'entraînement permet aussi une grande précision du contrôle de la position de la membrane, donc une très grande précision de débit.

Il n'est donc pas évident de satisfaire à l'ensemble de ces conditions dont certaines sont antagonistes.

Le but de la présente invention est d'apporter une solution qui permette de répondre, au moins en partie, aux conditions susmentionnées.

A cet effet, la présente invention a pour objet un dispositif de pompe doseuse à usage médical selon la revendication 1.

Les différentes particularités et les avantages de l'invention apparaîtront mieux à la lecture de la description qui suit de deux formes d'exécution du dispositif de pompe doseuse objet de l'invention données à titre d'exemples et illustrées schématiquement par les dessins annexés.

La figure 1 est une vue schématique d'ensemble du dispositif de pompe doseuse;

la figure 2 est un diagramme qui illustre une plage de travail typique de pompage avec une membrane de pompage plate;

la figure 3 est une vue en coupe d'une membrane de pompage selon l'invention destinée à être reliée à une enceinte de pompage;

la figure 4 est un diagramme pression-déplacement d'un dispositif de pompage utilisant la membrane de la figure 3;

la figure 5 est un diagramme comparatif pression-déplacement d'un dispositif de pompage qui utiliserait une membrane plate ayant les mêmes rapports de dimensions que celle de la figure 3;

la figure 6 est un diagramme de sensibilité à la pression de la membrane de la figure 3;

la figure 7 est un diagramme de sensibilité à la pression comparatif de la membrane plate de la figure 5,

la figure 8 est un schéma de la membrane du dispositif de pompage objet de l'invention montrant les dimensions caractéristiques dont les rapports dimensionnels seront discutés dans la description.

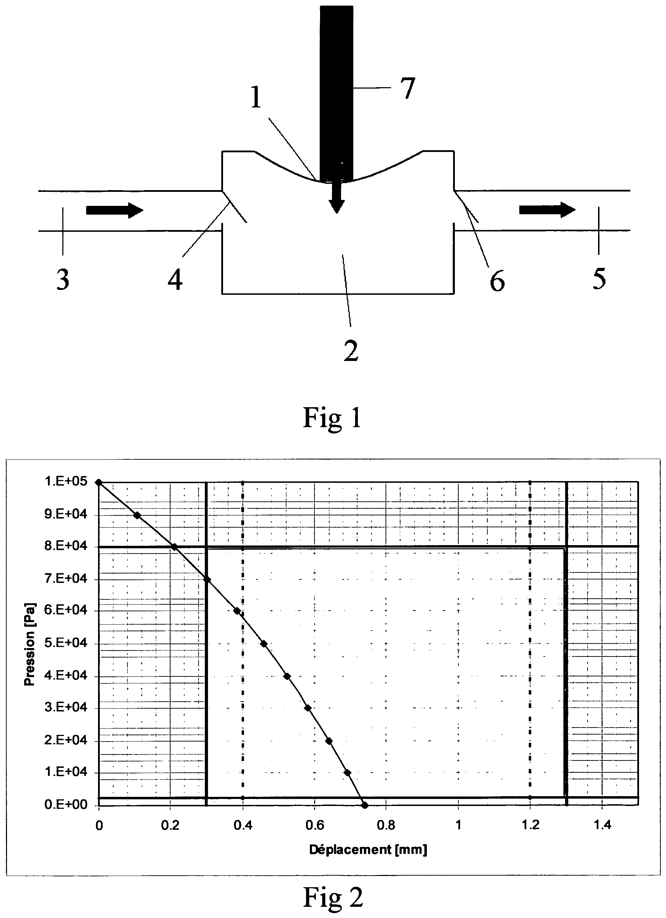

Le dispositif de pompe doseuse objet de l'invention est très schématiquement illustré par la figure 1 étant donné que c'est la membrane de pompage 1 déformable de manière élastique, sa géométrie et sa structure qui constituent l'élément novateur de cette invention.

Outre la membrane de pompage 1, ce dispositif comporte une enceinte de pompage 2 dans laquelle débouche un conduit amont 3 contrôlé par un clapet d'entrée 4, un conduit aval 5 étant lui contrôlé par un clapet de sortie 6. La membrane de pompage 1 est destinée à se déplacer entre une position limite de déplacement réduisant le volume de l'enceinte de pompage 2 en induisant une surpression apte à ouvrir le clapet de sortie 6 et une position limite de retour en induisant une dépression apte à fermer le clapet de sortie 6 et à ouvrir le clapet d'entrée 4.

L'objet de l'invention est plus particulièrement de déterminer comment réaliser une membrane permettant de satisfaire à un certain nombre de conditions.

Pour expulser le liquide de l'enceinte de pompage 2, un mécanisme d'entraînement 7, ici symbolisé par un poussoir, appuie sur la membrane de pompage 1 en direction de l'intérieur de l'enceinte de pompage. Lors de la phase d'aspiration de la pompe, c'est l'élasticité de la membrane qui produit la course de retour générant d'une part l'aspiration et d'autre part ramenant le mécanisme d'entraînement 7 à sa position de départ. Par conséquent un dimensionnement adéquat de la membrane 1 est primordial pour :

- Aspirer suffisamment (être capable de créer une dépression suffisante) lors du remplissage, pour lutter contre une dépression (perte de charge dans un conduit, hauteur de colonne d'eau, clapet avec seuil de pression, ...) et effectuer le remplissage suffisamment rapide de l'enceinte de pompage, et ceci sur toute sa plage de fonctionnement.
- Toutefois, l'aspiration ne doit pas créer une dépression trop forte, pour éviter des chocs dans les tuyaux, des effets de vaporisation du liquide contenu dans l'enceinte de pompage 1 par chute de pression au-delà de la pression de vapeur de ce liquide ou encore des effets de cavitation.
- Ne pas être trop sensible à la pression des conduits amont et aval afin de maintenir la précision des volumes incrémentaux pompés et par conséquent sur la précision du débit, indépendamment des pressions en amont et en aval du dispositif de pompage.

Nous allons examiner maintenant le dimensionnement de la membrane de pompage pour obtenir un remplissage adéquat.

Dans la suite de la description, on comparera le comportement d'une membrane annulaire de profil plat entourant un noyau central dont l'épaisseur est choisie pour qu'il ne se déforme aussi faiblement que possible, idéalement pas du tout, compte tenu des contraintes auxquelles il est soumis.

La caractéristique qui nous intéresse en premier lieu est la dépression que la membrane est capable de fournir en fonction du déplacement de son noyau central.

Le dimensionnement de la membrane de pompage 1 passe tout d'abord par la définition d'une plage de fonctionnement. Pour permettre à la membrane de pomper dès le début de sa course induisant une surpression, il est nécessaire de soumettre la membrane à une précontrainte comme on le verra par la suite.

Dans l'exemple retenu ici, la précontrainte de la membrane correspond à un déplacement de 0.4 mm à partir de sa position de repos, la plage de fonctionnement allant de 0.4 mm à 1.2 mm. Cette zone de fonctionnement est délimitée par deux lignes verticales en pointillé sur le diagramme de la figure 2. La pression dans l'enceinte de pompage ne doit pas descendre au-dessous de la pression de vaporisation de l'eau représentée par la ligne inférieure. Enfin, la plage de fonctionnement doit se situer à une pression inférieure à $-2.10^4$ Pa pour contrer la pression du liquide sur le côté amont du clapet d'entrée 4 ($1.10^4$ Pa), la hauteur de la colonne d'eau à aspirer ($5.10^3$ Pa) ainsi qu'un minimum de $5.10^3$ Pa pour ramener l'organe d'entraînement 7 à sa position de départ. La zone de travail de la membrane de pompage 1 est alors définie par le rectangle limité par les deux lignes verticales en pointillé et les deux lignes horizontales. Néanmoins, pour tenir compte des tolérances, une marge de 0.1 mm sur le déplacement de la membrane de pompage est prévue, comme illustré par les deux lignes verticales continues. La zone de travail possible est alors comprise entre 0.3 et 1.3 mm, illustrée par la figure 2.

Il ressort de cette figure que la membrane de pompage annulaire 1 doit présenter une rigidité telle que la pression absolue dans l'enceinte de pompage dans la plage de fonctionnement de la membrane située entre la position limite de déplacement et la position limite de retour soit comprise dans la plage suivante :

$$P_{atm} - \left( \left| \Delta P_{clapet\_in} \right| + \left| \Delta P_{\Delta h\_in} \right| + \left| \Delta P_{charge} \right| \right) \geq P > P_{vap}$$

où :

$P_{atm}$ = pression atmosphérique

$P_{vap}$ = pression de vapeur du fluide pompé

$\Delta P_{clapet\_in}$ = différence de pression entre l'amont et l'aval du clapet d'entrée pour son ouverture

$\Delta P_{\Delta h\_in}$ = différence de pression dans le conduit amont entre son extrémité distale et le côté amont du clapet d'entrée dû au poids de la colonne de fluide

∆Pcharge = perte de charge dans le conduit amont pour le débit souhaité.

La figure 2 représente encore la courbe pression déplacement de la membrane plate susmentionnée. On constate que l'on arrive à une situation où la dépression générée par la membrane en déplacement x > 0.7 mm est nettement trop forte, ce qui pourrait provoquer des chocs (ondes de pression dans le conduit amont 3), des phénomènes d'ébullition par chute de pression et/ou de cavitation. En d'autres termes, la pente qui est d'environs -1.10$^4$ Pa/0.1 mm est trop forte. De manière inverse, si l'on cherche à diminuer la pente au maximum, on risque d'être à une pression égale à -2.10$^4$ Pa à la position de précontrainte de la membrane correspondant à un déplacement de 0,3 mm et de ne pas pouvoir finir l'aspiration, autrement dit d'aspirer moins que le volume de pompage de la pompe.

Afin de remédier à ce problème, on cherche à réaliser une membrane qui travaillerait à pression la plus constante possible sur la plage de fonctionnement. Ceci permettrait d'une part de travailler dans la zone de fonctionnement admissible (condition indispensable) et d'autre part d'adoucir les chocs dans le conduit amont 3 et d'éviter la vaporisation du liquide ou la cavitation.

Afin de s'approcher d'une telle membrane on a examiné une géométrie de membrane dont le profil à l'état de repos est tronconique, qui résulte donc du déplacement du noyau central rigide de la membrane plane dans un plan parallèle, la partie annulaire de la membrane étant alors conique à l'état de repos. Les efforts qui résultent du déplacement du noyau central rigide parallèlement à son plan d'une telle membrane tronconique se décomposent en efforts de traction compression et en efforts de flexion de la partie annulaire flexible.

On distingue trois comportements au fur et à mesure du déplacement du noyau central parallèlement à son plan:

- La membrane travaille principalement en compression, la flexion est secondaire.
- La compression diminue avec le déplacement du noyau jusqu'à un seuil où la membrane flambe et où les efforts de compression sont relaxés. Les efforts de flexion augmentent.
- Les efforts de compression sont totalement relaxés et la membrane travaille en traction. Les efforts de flexion continuent d'augmenter avec le déplacement.

Ainsi une membrane de forme tronconique possède une caractéristique pression déplacement en forme de vague résultant du phénomène de flambage.

Suivant l'épaisseur d'une telle membrane on distingue deux comportements différents:

- Lorsque l'épaisseur est très faible et que l'angle du cône est prononcé, la membrane flambe fortement, elle se comporte comme une membrane bi-stable dont la caractéristique pression déplacement forme un S très prononcé. Dans ce cas ce sont les efforts de traction compression qui dominent
- Si l'épaisseur est importante et la pente faible, le phénomène de flambage n'apparaît pas ou faiblement, il s'agit d'une membrane stable dont la caractéristique pression déplacement ne forme pas de S. C'est les efforts de flexion qui dominent.

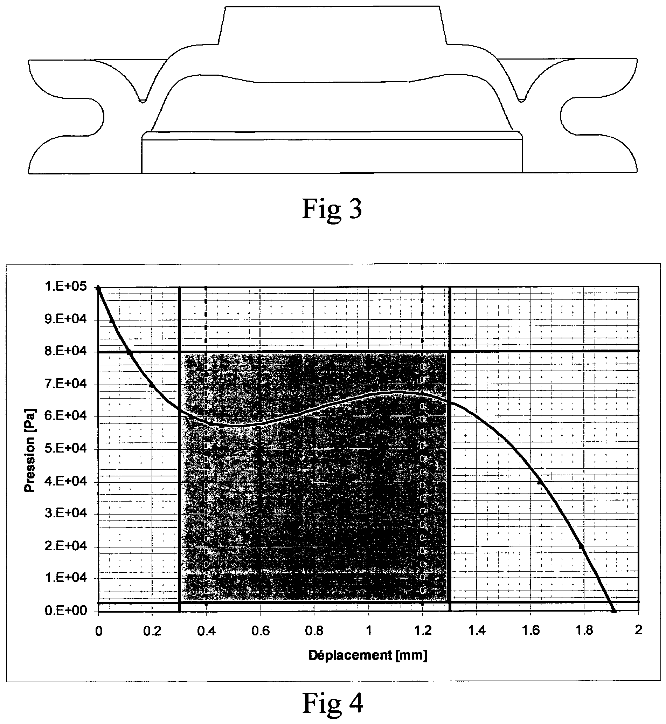

Une combinaison de ces deux solutions extrêmes telle que celle illustrée par la figure 3 donne une courbe intéressante. La figure 4 illustre la courbe pression déplacement d'une membrane annulaire du type de celle de la figure 3 objet de l'invention dont la géométrie se caractérise essentiellement par un profil annulaire convexe bombé vers l'extérieur de l'enceinte de pompage. Dans cette forme d'exécution, le bord externe de la membrane 1 est solidaire d'un élément de liaison annulaire 1b qui présente une gorge annulaire le destinée à permettre sa fixation à la paroi de l'enceinte de pompage 2.

Le type de profil de membrane de pompage 1 de la figure 3, rend possible d'ajuster la distance entre le plan du noyau central la de cette membrane 1 et le plan passant par le bord externe de la membrane annulaire 1, où elle est reliée à l'enceinte de pompage par l'élément de liaison annulaire 1b. On peut aussi varier le diamètre du noyau central la. Ainsi, en faisant varier différents paramètres géométriques de la membrane de pompage 1, on peut modifier l'amplitude de la courbe pression déplacement de la figure 4. Suivant les caractéristiques de la membrane 1 de la figure 3 on relève les effets suivants :

- Plus l'angle du cône est aigu, plus les efforts de traction compression dominent et plus la caractéristique pression déplacement de la membrane prend une forme en S prononcée.
- Plus la membrane annulaire 1 est épaisse, plus les efforts de flexion dominent :
- moins la caractéristique pression déplacement prend une forme en S prononcée
- plus la pente de la caractéristique pression déplacement est forte
- moins la plage de fonctionnement de la membrane peut être large
- Plus la membrane annulaire 1 présente une forme bombée, plus les efforts de flexion dominent et moins la caractéristique pression déplacement prend une forme en S prononcée.

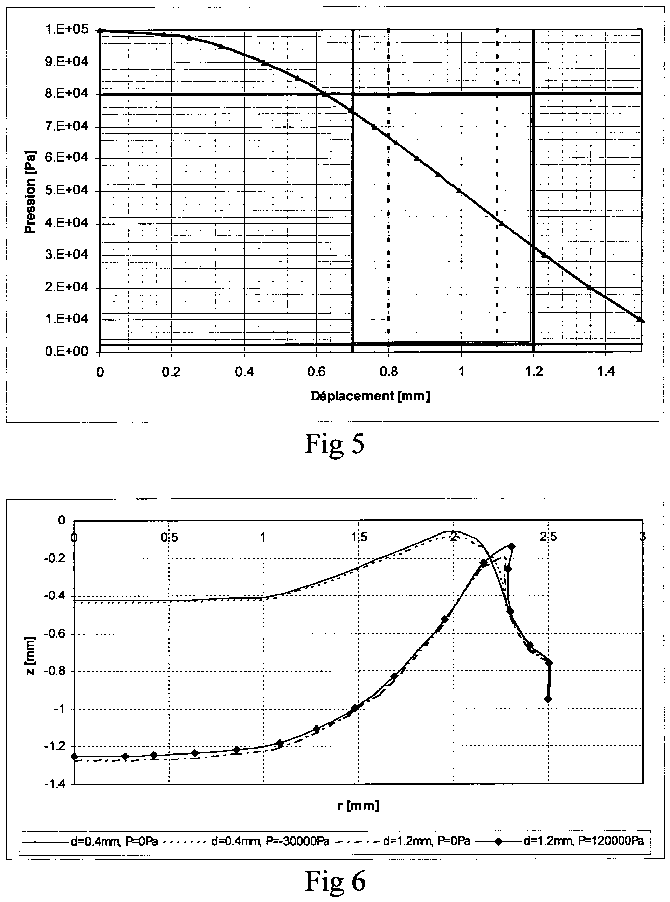

Une manière de réduire la sensibilité de la membrane de pompage annulaire conique à la pression serait de réduire la plage de fonctionnement et d'augmenter la pré-contrainte, par exemple en passant d'une plage de fonctionnement de 0.3 à 1.3 mm à une plage de fonctionnement 0.7 à 1.2 mm. On pourrait, dans ce cas, réduire la rigidité de la membrane dont la pente serait alors typiquement de $1.10^4$ Pa/0.2 mm qui travaillerait sur une plage rétrécie, telle que celle de la figure 5.

Néanmoins une telle membrane comporterait les inconvénients suivants :

- La précontrainte de la membrane est augmentée, en sorte que les efforts dans la membrane sont plus importants.
- Le débit du dispositif de pompage est réduit dans le même rapport que la plage de fonctionnement.
- Un tel dispositif est beaucoup plus sensible à la pression du fait que l'on réduit l'épaisseur de la membrane afin de réduire la pente de la courbe pression déplacement, en sorte que l'on augmente fortement la sensibilité d'une telle membrane à la pression, ce qui réduit de manière significative la précision du débit.

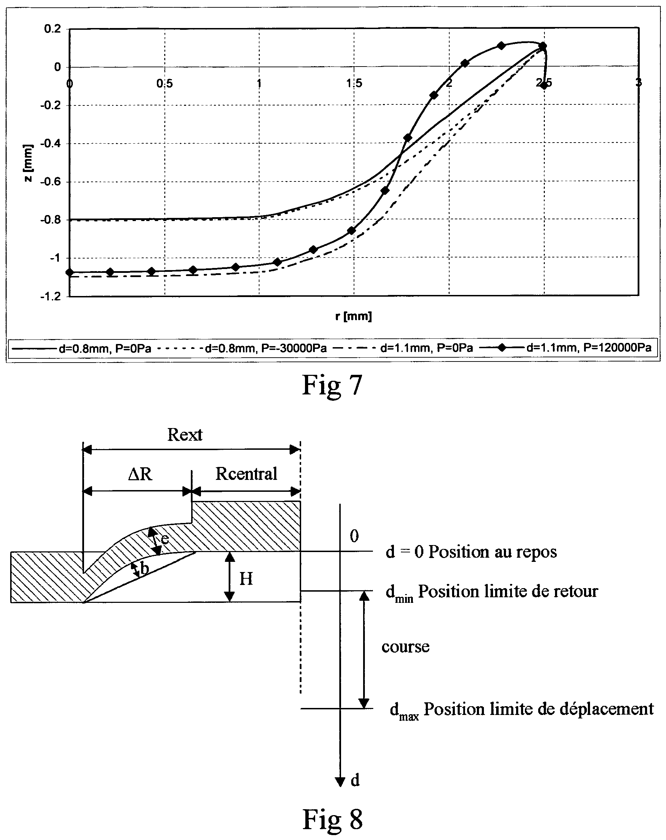

Afin d'illustrer cette sensibilité à la pression, les figures 6 et 7 indiquent la sensibilité à la pression de la membrane de la figure 3, comparée avec une membrane annulaire plane ayant les mêmes rapports de diamètres interne/externe: Dans chaque cas, les diagrammes illustrent le noyau central 1a de la membrane dans deux positions parallèles à son plan par rapport à son bord externe fixé à l'enceinte de pompage.

La membrane est illustrée dans une première position limite sous précontrainte du noyau central 1a, correspondant à la position limite de retour de la membrane, une fois sous pression nulle et une fois sous une pression de $-3.10^4$ Pa.

La membrane est illustrée en position limite de déplacement correspondant à la fin de la course d'expulsion du liquide de l'enceinte de pompage, une fois à pression nulle et une fois à une pression de $1,2.10^5$ Pa.

On constate sur la figure 6 que la membrane selon la présente invention est peu sensible à la pression. En effet on ne voit que peu de différence entre la courbe en trait continu et celle en pointillé où elle est soumise à une dépression de $-3.10^4$ Pa ou encore entre les courbes en traits mixtes et celle en traits-croix où elle est soumise à une pression de $1,2.10^5$ Pa. Par contre sur la figure 7, on constate, dans les mêmes conditions de mesure, un grand effet de la pression sur la forme de la membrane.

Les différents paramètres dimensionnels préférentiels de la membrane pour obtenir les effets souhaités en ce qui concerne la caractéristique pression déplacement, ainsi que la réduction de sensibilité à la pression sont indiqués sur la figure 8 qui représente une coupe de la moitié de la membrane 1 de son centre à sa périphérie.

Les paramètres dimensionnels de cette membrane contribuant à l'obtention des caractéristiques susmentionnées sont les suivants et doivent être contenus dans les intervalles suivants :

$$H > 0$$

$$0.1 \leq \frac{\Delta R}{R_{ext}} \leq 0.9$$

$$0.05 \leq \frac{\Delta R}{d_{max}} \leq 5$$

$$0.01 \leq \frac{e}{\Delta R} \leq 1$$

$$0 < \frac{b}{e} < 10$$

pour un matériau élastique dont le module d'élasticité est compris entre $0.1$ MPa $\leq E \leq 100$ MPa. Ce sont aussi les paramètres $b$ et $e$ qui permettent d'influencer la courbe pression déplacement.

L'augmentation de $\Delta R/R_{ext}$ entraîne une réduction de la compression / relaxation de compression, une réduction de la traction. Cette augmentation de $\Delta R/R_{ext}$ a aussi pour effet de réduire l'amplitude de pression de la courbe pression déplacement (voir figure 4) en la rendant plus plate.

L'augmentation de H/$R_{ext}$ a pour effet d'augmenter la compression, relaxation de compression, d'augmenter la traction, d'augmenter de course possible de la membrane et d'augmenter l'amplitude de pression de la courbe pression déplacement.

L'augmentation de e/$\Delta$R augmente les effets de flexion et la rigidité et réduit l'amplitude de la courbe pression déplacement (figure 4) en l'aplatissant.

Le tableau ci-dessous donne à titre d'exemple les paramètres dimensionnels et de fréquence/débit de trois dispositifs de pompage objets de la présente invention.

| Volume | [$\mu$L] | 75 | 10 | 1 |
|---|---|---|---|---|
| $E_{membrane}$ | [MPa] | 3.5 | 3.5 | 3.5 |
| $R_{ext}$ | [mm] | 6.0 | 2.5 | 1.0 |
| $R_{central}$ | [mm] | 3.5 | 1.5 | 0.6 |
| H | [mm] | 0.5 | 0.4 | 0.3 |
| Course | [mm] | 1 | 0.8 | 0.5 |
| $Fréq_{min}$ | [Hz] | 0.004 | 0.003 | 0.003 |
| $Fréq_{max}$ | [Hz] | 4 | 15 | 30 |
| $Débit_{min}$ | [mL/h] | 1 | 0.1 | 0.01 |
| $Débit_{max}$ | [mL/h] | 1000 | 500 | 100 |

Avantageusement, la membrane est en silicone. Elle pourrait aussi être en polyuréthane ou en EPDM. L'enceinte de pompage étant avantageusement en polycarbonate et la membrane de la figure 3 étant soudée.

## Revendications

**1.** Dispositif de pompe doseuse à usage médical comprenant une enceinte de pompage (2) un organe d'entraînement (7), une membrane de pompage (1) annulaire dont le bord externe est solidaire de ladite enceinte de pompage et dont le bord interne est solidaire d'une partie centrale d'entraînement plus rigide que ladite membrane, susceptible d'être déplacée par l'organe d'entraînement (7) parallèlement à elle-même entre une position limite de retour et une position limite de déplacement dans sa course d'expulsion et inversement dans sa course d'aspiration, ces deux positions se situant de part et d'autre du plan contenant le bord externe de ladite membrane annulaire, ladite enceinte de pompage comprenant un clapet d'entrée (4) et un clapet de sortie (6) ouverts par une dépression, respectivement par une surpression dans l'enceinte de pompage, consécutives aux déplacements de ladite membrane de pompage annulaire, la course d'aspiration de ladite membrane résultant de l'énergie accumulée par la déformation élastique de la membrane lors de sa course d'expulsion, dans laquelle ladite membrane de pompage annulaire présente, dans ladite position limite de retour, un profil annulaire convexe bombé vers l'extérieur de ladite enceinte de pompage, vers l'organe d'entraînement (7), pour que sa course d'expulsion se traduise essentiellement par la déformation en flexion génératrice de l'énergie de la course d'aspiration et par la compression annulaire/relaxation de compression dudit profil annulaire convexe à chaque course aller de ladite membrane, la rigidité de la membrane étant choisie pour que la pression absolue dans l'enceinte de pompage dans la plage de fonctionnement de la membrane située entre-la position limite de déplacement et la position limite de retour soit comprise dans la plage suivante :

$$P_{atm} - \left( \left| \Delta P_{clapet\_in} \right| + \left| \Delta P_{\Delta h\_in} \right| + \left| \Delta P_{charge} \right| \right) \geq P > P_{vap}$$

où :

$P_{atm}$ = pression atmosphérique
$P_{vap}$ = pression de vapeur du fluide pompé
$\Delta P_{clapet\_in}$ = différence de pression entre l'amont et
l'aval du clapet d'entrée pour son ouverture
$\Delta P_{Ah\_in}$ = différence de pression dans le conduit amont entre son extrémité distale et le côté amont du clapet d'entrée dû au poids de la colonne de fluide
$\Delta P_{charge}$ = perte de charge dans le conduit amont pour le débit souhaité,
la membrane étant soumise à une précontrainte dans sa position de retour extrême,

**caractérisée en ce que** la membrane a une forme tronconique à l'état de repos et **en ce que** son déplacement de sa position de retour extrême à sa position de déplacement extrême provoque un flambage se traduisant par une caractéristique pression-déplacement en forme de S.

2. Dispositif de pompage selon la revendication 1, dans lequel ladite membrane est en silicone.

3. Dispositif selon l'une des revendications précédentes dans lequel le rapport entre la largeur radiale ∆R de la partie annulaire de la membrane de pompage et son rayon extérieur est compris dans la plage :

$$0.1 \le \frac{\Delta R}{R_{ext}} \le 0.9$$

4. Dispositif selon l'une des revendications précédentes dans lequel le rapport entre la largeur radiale ∆R de la partie annulaire de la membrane annulaire de pompage et la longueur de la course de la membrane entre sa position limite de retour et sa position limite de déplacement est compris dans la plage :

$$0.05 \le \frac{\Delta R}{d_{max}} \le 5$$

5. Dispositif selon l'une des revendications précédentes dans lequel le rapport entre l'épaisseur e de la membrane annulaire de pompage et la largeur radiale ∆R de sa partie annulaire est compris dans la plage :

$$0.01 \le \frac{e}{\Delta R} \le 1$$

6. Dispositif selon l'une des revendications précédentes dans lequel le rapport entre la distance maximum entre la corde qui sous-tend le côté concave de la partie annulaire de la membrane et l'épaisseur e de cette membrane est compris dans la plage :

$$0 < \frac{b}{e} < 10$$

pour un matériau élastique dont le module d'élasticité E est compris entre **0.1$MPa \le E \le 100MPa$**.

7. Dispositif selon la revendication 1, dans lequel ladite plage de fonctionnement se situe entre 0,7 et 1,2 mm, avec une rigidité de la membrane dont la pente est typiquement de $1.10^4$ Pa/0,2 mm.

8. Dispositif selon l'une des revendications précédentes dont l'enceinte de pompage est en polycarbonate.

9. Dispositif selon l'une des revendications précédentes dont la membrane de pompage annulaire est un élément surmoulé à l'enceinte de pompage.


**Patentansprüche**

1. Dosierpumpenvorrichtung für medizinische Zwecke mit einem Pumpraum (2), einem Antriebsorgan (7), einer ringförmigen Pumpmembran (1), deren Außenrand fest mit dem Pumpraum verbunden ist und deren Innenrand fest mit einem zentralen Antriebsteil verbunden ist, das steifer ist als die Membran und dazu geeignet ist, durch das Antriebsorgan (7) parallel zu sich selbst zwischen einer Rückkehr-Grenzstellung und einer Verschiebe-Grenzstellung in seinem Ausstoßhub und umgekehrt in seinem Ansaughub verschoben zu werden, wobei diese beiden Stellungen beiderseits einer Ebene liegen, die den Außenrand der genannten ringförmigen Membran enthält, wobei der genannte Pumpraum ein Einlassventil (4) und ein Auslassventil (6) aufweist, die durch einen Unterdruck bzw. durch einen Überdruck im Pumpraum geöffnet werden, die aus Verschiebungen der genannten ringförmigen Pumpmembran folgen, wobei der Ansaughub der genannten Membran aus der Energie resultiert, die durch die elastische Verformung der Membran während ihres Ausstoßhubs gespeichert wird, wobei die genannte ringförmige Pumpmembran in der genannten Rückkehr-Grenzstellung ein konvexes ringförmiges Profil aufweist, das nach außen aus dem genannten Pumpraum in Richtung des Antriebsorgans (7) gewölbt ist, so dass ihr Ausstoßhub im Wesentlichen in einer Energie generierenden Biegeverformung des Ansaughubs und in der ringförmigen Kompression/Kompressionserholung des konvexen Ringprofils bei jedem Hinhub der Membran resultiert, wobei die Steifigkeit der Membran so gewählt ist, dass der absolute Druck in der Pumpenkammer in dem Betriebsbereich der Membran zwischen der Verschiebe-Grenzstellung und der Rückkehr-Grenzstellung innerhalb des folgenden Bereichs liegt:

$$P_{atm} - \left( \left| \Delta P_{clapet\_in} \right| + \left| \Delta P_{\Delta h\_in} \right| + \left| \Delta P_{ch\arg e} \right| \right) \geq P > P_{vap}$$

wobei :

$P_{atm}$ = atmosphärischer Druck
$P_{vap}$ = Dampfdruck des gepumpten Fluids.
$\Delta P_{clapet\_in}$ = Druckdifferenz zwischen der stromaufwärtigen und der stromabwärtigen Seite des Einlassventils zum Öffnen desselben.
$\Delta P_{Ah\_in}$ = Druckdifferenz in der stromaufwärts gelegenen Leitung zwischen ihrem distalen Ende und der stromaufwärts gelegenen Seite des Einlassventils aufgrund des Gewichts der Flüssigkeitssäule.
$\Delta P_{charge}$ = Druckverlust in der stromaufwärts gelegenen Leitung für den gewünschten Durchfluss,
wobei die Membran in ihrer extremen Rückkehrstellung einer Vorspannung unterliegt,
**dadurch gekennzeichnet, dass** die Membran im Ruhezustand eine kegelstumpfförmige Form hat und dass ihre Bewegung von ihrer extremen Rückkehrstellung zu ihrer extremen Verschiebestellung eine Knickung hervorruft, die durch eine charakteristische Druck-Verschiebung in Form eines S zum Ausdruck kommt.

2. Pumpvorrichtung nach Anspruch 1, wobei die Membran aus Silikon ist.

3. Vorrichtung nach einem der vorhergehenden Ansprüche, wobei das Verhältnis zwischen der radialen Breite ∆R des ringförmigen Teils der Pumpmembran und ihrem Außenradius im Bereich liegt:

$$0.1 \leq \frac{\Delta R}{R_{ext}} \leq 0.9$$

4. Vorrichtung nach einem der vorhergehenden Ansprüche, wobei das Verhältnis zwischen der radialen Breite ∆R des ringförmigen Teils der ringförmigen Pumpmembran und der Länge des Hubs der Membran zwischen ihrer Rückkehr-Grenzstellung und ihrer Verschiebe-Grenzposition im Bereich liegt:

$$0.05 \leq \frac{\Delta R}{d_{\max}} \leq 5$$

5. Vorrichtung nach einem der vorhergehenden Ansprüche, wobei das Verhältnis zwischen der Dicke e der ringförmigen Pumpmembran und der radialen Breite ∆R ihres ringförmigen Teils im Bereich liegt:

$$0.01 \leq \frac{e}{\Delta R} \leq 1$$

6. Vorrichtung nach einem der vorhergehenden Ansprüche, wobei das Verhältnis zwischen dem maximalen Abstand zwischen der Sehne, die die konkave Seite des ringförmigen Teils der Membran unterspannt, und der Dicke e dieser Membran im Bereich liegt :

$$0 < \frac{b}{e} < 10$$

für ein elastisches Material, dessen Elastizitätsmodul E zwischen $0{,}1\,\text{MPa} \leq E \leq 100\,\text{MPa}$ liegt.

7. Vorrichtung nach Anspruch 1, wobei der genannte Betriebsbereich zwischen 0,7 und 1,2 mm liegt, mit einer Membransteifigkeit, deren Steigung typischerweise $1.10^4$ Pa/0,2 mm beträgt.

8. Vorrichtung nach einem der vorhergehenden Ansprüche, wobei deren Pumpenkammer aus Polycarbonat ist.

9. Vorrichtung nach einem der vorhergehenden Ansprüche, wobei deren ringförmige Pumpmembran ein Element ist, das an das Pumpraum angegossen ist.

## Claims

1. Dosing pump device for medical use comprising a pumping chamber (2), a drive member (7), an annular pumping membrane (1), the outer edge of which is integral with the said pumping chamber and the inner edge of which is integral with a central drive part which is more rigid than the said membrane, capable of being displaced by the drive member (7) parallel to itself between a return limit position and a displacement limit position in its expulsion stroke and vice versa in its suction stroke, these two positions being located on either side of the plane containing the outer edge of the said annular membrane,
said pumping chamber comprising an inlet valve (4) and an outlet valve (6) opened by a depression, respectively by an overpressure in the pumping chamber, consecutive to the displacements of said annular pumping membrane, the suction stroke of said membrane resulting from the energy accumulated by the elastic deformation of the membrane during its expulsion stroke, wherein in said return limit position, said annular pumping membrane has a convex annular profile curved towards the outside of said pumping chamber, towards the drive member (7), so that its expulsion stroke results essentially in the energy-generating bending deformation of the suction stroke and in the annular compression/release of compression of the said convex annular profile on each outward stroke of the said membrane, the rigidity of the membrane being chosen so that the absolute pressure in the pumping chamber in the operating range of the membrane situated between the limit position of displacement and the limit position of return lies within the following range :

$$P_{atm} - \left( \left| \Delta P_{clapet\_in} \right| + \left| \Delta P_{\Delta h\_in} \right| + \left| \Delta P_{ch\,arg\,e} \right| \right) \geq P > P_{vap}$$

where :

$P_{atm}$ = atmospheric pressure
$P_{vap}$= vapour pressure of the pumped fluid
$\Delta P_{clapet\_in}$ = pressure difference between the upstream and downstream sides of the inlet valve for its opening
$\Delta P_{Ah\_in}$= pressure difference in the upstream pipe between its distal end and the upstream side of the inlet valve due to the weight of the fluid column
$\Delta P_{charge}$ = pressure drop in the upstream pipe for the desired flow rate,
the membrane being subject to prestress in its extreme return position,
**characterised in that** the membrane has a truncated cone shape in the resting state and **in that** its displacement from its extreme return position to its extreme displacement position causes buckling resulting in an S-shaped pressure-displacement characteristic.

2. A pumping device as claimed in claim 1, wherein said membrane is made of silicone.

3. A device according to any of the preceding claims
wherein the ratio between the radial width $\Delta R$ of the annular portion of the pumping membrane and its outer radius is within the range:

$$0.1 \le \frac{\Delta R}{R_{ext}} \le 0.9$$

4. A device according to any of the preceding claims in which the ratio between the radial width $\Delta R$ of the annular portion of the annular pumping membrane and the length of the stroke of the membrane between its return limit position and its displacement limit position is in the range:

$$0.05 \le \frac{\Delta R}{d_{max}} \le 5$$

5. A device according to any of the preceding claims wherein the ratio between the thickness e of the annular pumping membrane and the radial width $\Delta R$ of its annular portion is in the range:

$$0.01 \le \frac{e}{\Delta R} \le 1$$

6. A device according to any of the preceding claims wherein the ratio between the maximum distance between the chord subtending the concave side of the annular portion of the membrane and the thickness e of that membrane is in the range :

$$0 < \frac{b}{e} < 10$$

for an elastic material with a modulus of elasticity E between 0.1 MPa <= E <= 100 MPa.

7. A device according to claim 1, wherein said operating range is between 0.7 and 1.2 mm, with a membrane stiffness whose slope is typically $1.10^{-4}$ Pa/0.2 mm.

8. A device according to one of the preceding claims, the pumping chamber of which is made of polycarbonate.

9. A device according to one of the preceding claims, the annular pumping membrane of which is an element overmoulded to the pumping chamber.

Fig 1

Fig 2

Fig 3

Fig 4

Fig 5

Fig 6

Fig 7

Fig 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2046599 A5